# EUROPEAN PATENT SPECIFICATION

(11) **EP 2 446 847 B1**
(45) Date of publication and mention of the grant of the patent: **04.05.2016**
(21) Application number: 11765445.9
(22) Date of filing: 25.03.2011
(51) Int. Cl.: B06B 1/02, A61B 17/32

(54) **ULTRASONIC SURGERY SYSTEM**
ULTRASCHALL-OPERATIONSSYSTEM
SYSTÈME DE CHIRURGIE ULTRASONORE

(30) Priority: 09.04.2010 US 322510 P
(43) Date of publication of application: 02.05.2012
(73) Proprietor: Olympus Corporation, Shibuya-ku Tokyo (JP)
(72) Inventor: SANAI, Hideo, Tokyo 151-0072 (JP)
(74) Representative: Gunzelmann, Rainer
(86) International application number: PCT/JP2011/057357
(87) International publication number: WO 2011/125544

(56) References cited:
- EP-A2- 0 135 907
- WO-A2-2009/018409
- JP-A- 2 283 362
- JP-A- 3 075 000
- JP-A- 2000 060 866
- JP-A- 2008 055 151
- US-A- 5 087 850
- US-A1- 2005 020 967

## Description

### Technical Field

The present invention relates to an ultrasound operation system and a surgical treatment instrument, and particularly relates to an ultrasound operation system and a surgical treatment instrument which perform amplitude regulation of an ultrasound transducer.

### Background Art

Conventionally, as an ultrasound operation system for a surgical operation which applies ultrasound vibration, an ultrasound operation system has been developed, which dissects or coagulates a living tissue by using an ultrasound probe which is ultrasound-vibrated. Ultrasound vibration in the ultrasound operation system is provided by driving control of an ultrasound transducer incorporated in a hand piece, and a distal end portion of the ultrasound probe vibrates with predetermined amplitude.

However, a value of the amplitude varies depending on a component variation or an assembly variation of an ultrasound transducer, or a component variation or an assembly variation of an ultrasound probe. There is such a problem that, when the value of the amplitude is large, stress of the ultrasound probe increases. Suppressing such a component variation or an assembly variation of the ultrasound transducer, or a variation of components themselves of the ultrasound probe or an assembly variation of the ultrasound probe will lead to an increase in cost.

Further, when a hand piece is in a shape of scissors, a grasping force amount of a handle varies depending on a component variation or an assembly variation. There are such problems that, when the grasping force amount is small, a dissection speed decreases, and when the grasping force amount is large, wear promotion of a Teflon pad and stress of the ultrasound probe increase. Suppressing such a variation of a component itself which is related to the grasping force or an assembly variation will lead to an increase in cost. In addition, also in the case where the hand piece is in a shape of scissors, the value of the amplitude varies depending on the above-described component variation or the assembly variation of the ultrasound transducer, or the component variation or the assembly variation of the ultrasound probe. There are such problems that, when the value of the amplitude is small, a dissection speed decreases, and when the value of the amplitude is large, the vascular withstanding pressure ability is reduced and wear promotion of a Teflon pad increases.

Furthermore, a hardness of a site to be treated which a surgeon treats differs in accordance with a site. There are such problems that, when the site to be treated is a thin tissue, a dissection speed decreases, whereas when the site to be treated is a hard tissue, the stress of an ultrasound probe increases and main apparatus cannot output ultrasound in some cases. In view of usability, it is not preferable that the surgeon can treat only a specific site to be treated, that is, an ordinary tissue.

Therefore, for example, Japanese Patent Application Laid-Open Publication No. 2005-27907 proposes an ultrasound operation system which detects impedance at a drive time of an ultrasound transducer, and performs control of a drive signal which is supplied to the ultrasound transducer.

However, since the ultrasound operation system proposed in the above-described Japanese Patent Application Laid-Open Publication No. 2005-27907 calculates impedance at the time of driving of the ultrasound transducer which corresponds to a mechanical load exerted on the ultrasound probe, and feedbacks the impedance to a main apparatus, to control an electric current to be supplied to the ultrasound transducer in the control section of the main apparatus, there is a need for providing a control program and a control section for performing feedback control. Therefore, there is such a problem that the above-proposed ultrasound operation system requires a control program and a control section for performing feedback control, which increases a cost.

Therefore, an object of the present invention is to provide an ultrasound operation system capable of controlling an electric current flowing into an ultrasound transducer without a need for providing a control program and a control section for performing feedback control.

US 5,087,850 teaches how to cope with an ultrasonic transducer apparatus in which the ultrasonic probe can be substituted.

### Disclosure of Invention

### Means for Solving the Problem

The present invention is defined in claim 1.

According to one aspect of the present invention, it is possible to provide an ultrasound operation system which includes a drive current generating portion, a surgical treatment instrument including an object to be driven including an ultrasound transducer which is driven by the drive current generating portion, and a treatment portion which is connected to the ultrasound transducer to perform treatment of a living tissue by an ultrasound vibration generated in the ultrasound transducer, and an amplitude regulating portion which is provided between the drive current generating portion and the object to be driven, and performs regulation of an amplitude of the ultrasound transducer by diverting a current which flows into the object to be driven from the drive current generating portion in accordance with a state of a load exerted on the treatment portion and regulating an amount of a current which flows into the object to be driven.

Further, according to another aspect of the present invention, it is possible to provide a surgical treatment instrument which includes an object to be driven including an ultrasound transducer, and an amplitude regulating portion which is provided in parallel with the object to be driven, diverts a current which flows into the object to be driven and is for driving the object to be driven, and performs regulation of an amplitude of the ultrasound transducer.

### Brief Description of the Drawings

[Fig. 1] Fig. 1 is a view showing a configuration of an ultrasound operation system according to an embodiment of the present invention.
[Fig. 2] Fig. 2 is a diagram showing an equalizing circuit of the ultrasound operation system before ultrasound output of the present embodiment.
[Fig. 3] Fig. 3 is a diagram showing the equalizing circuit of the ultrasound operation system during ultrasound output of the present embodiment.
[Fig. 4] Fig. 4 is an explanatory diagram for explaining a relationship of a non-inductive resistance value and a current which is supplied to an ultrasound transducer.
[Fig. 5] Fig. 5 is an explanatory diagram for explaining a relationship of a load on a treatment portion and a current which is supplied to the ultrasound transducer.
[Fig. 6] Fig. 6 is an explanatory diagram for explaining a relationship of the treatment portion amplitude value and an oscillation efficiency of a main apparatus.
[Fig. 7] Fig. 7 is an explanatory diagram for explaining a relationship of the treatment portion amplitude value and a dissection time.
[Fig. 8] Fig. 8 is an explanatory diagram for explaining a relationship of the treatment portion amplitude value and a vascular withstanding pressure average value.
[Fig. 9] Fig. 9 is an explanatory diagram for explaining a relationship of the treatment portion amplitude value and a number of dissection times.
[Fig. 10] Fig. 10 is a diagram showing an equalizing circuit of a conventional ultrasound operation system.

### Best Mode for Carrying Out the Invention

### (Entire Configuration)

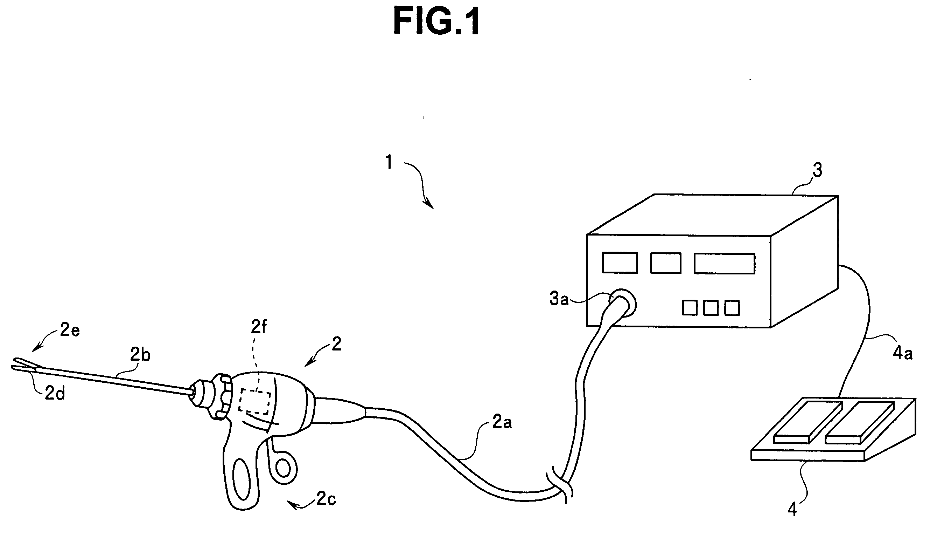

Fig. 1 is a view showing a configuration of an ultrasound operation system according to an embodiment of the present invention. An ultrasound operation system 1 is configured by including a hand piece 2, a main apparatus 3 that is an output control apparatus, and a foot switch 4.

The hand piece 2 is a surgical treatment instrument capable of outputting ultrasound. The hand piece 2 is connected to the main apparatus 3 via a cable 2a which is attachable and detachable. The hand piece 2 has an insertion portion 2b and a handle portion 2c. Further, the hand piece 2 contains an object to be driven including an ultrasound transducer 2f. The ultrasound transducer 2f has a vibration block which is configured into a cylindrical shape in such a manner that a plurality of piezoelectric elements each formed into a donut shape are stacked to sandwich a plurality of annular electrodes which are disposed between adjacent piezoelectric elements. Further, a bolt is penetrated through through-holes in centers of the piezoelectric elements and the electrodes which are stacked, and the bolt is screwed into a horn portion, whereby the ultrasound transducer 2f is configured so that the piezoelectric elements and the electrodes are firmly brought into close contact with one another. The ultrasound transducer 2f is a unit of a Langevin type bolted ultrasound transducer.

Furthermore, the hand piece 2 has an ultrasound probe 2d, and at a distal end side of the ultrasound probe 2d, a treatment portion 2e is formed by a distal end portion of the ultrasound probe 2d and a movable piece which is openable and closable with respect to the distal end portion.

The main apparatus 3 supplies a drive signal for ultrasound output to the ultrasound transducer 2f contained in the hand piece 2. The ultrasound transducer 2f ultrasound-vibrates by being supplied with the drive signal. The ultrasound vibration is transmitted to the distal end portion of the ultrasound probe 2d via the ultrasound probe 2d in the insertion portion 2b. The hand piece 2 can generate a friction heat in a living tissue of an object to be treated, and can perform treatment such as coagulation, dissection, and the like.

The foot switch 4 is connected to the main apparatus 3 via a cable 4a. The foot switch 4 is a switch for turning on or off ultrasound output at the time of ultrasound output.

A surgeon pulls a wire (not illustrated) which is inserted in the insertion portion 2b by putting a finger on the handle portion 2c and performing an opening and closing operation to open and close a movable piece in the treatment portion 2e to be able to grasp a living tissue which is the object to be treated. Further, the surgeon can perform, for example, a laparoscopic surgical operation with the hand piece 2 in one hand, and another treatment instrument in the other hand.

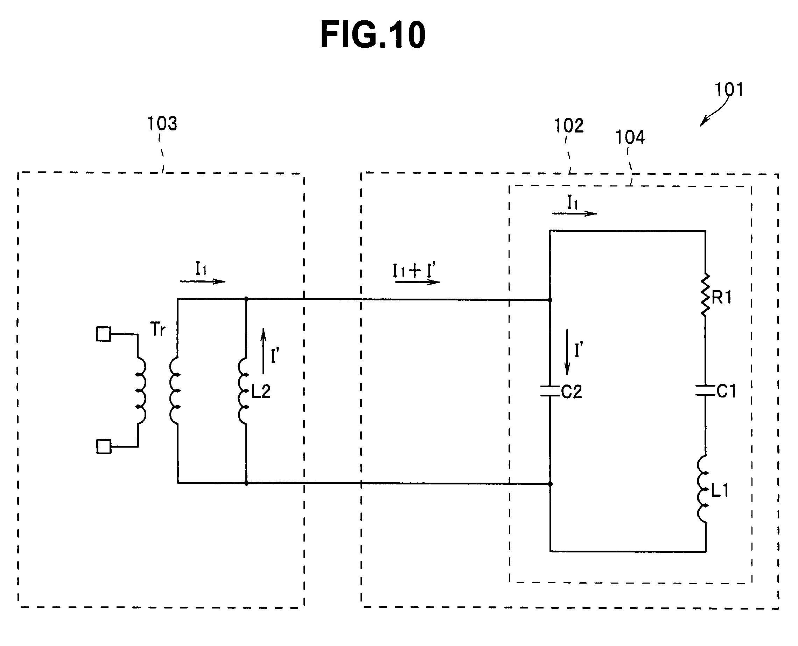

Here, an equalizing circuit of a conventional ultrasound operation system will be described. Fig. 10 is a diagram showing the equalizing circuit of the conventional ultrasound operation system.

As shown in Fig. 10, an ultrasound transducer 104 provided in a hand piece 102 of an ultrasound operation system 101 is configured by a series resonance circuit in which a resistor R1, a capacitor C1 and a coil L1 are connected in series, and a capacitor C2 connected in parallel to the series circuit.

A main apparatus 103 of the ultrasound operation system 101 is configured by an output transformer Tr, and a coil L2 which is connected to the output transformer Tr in parallel. A signal which is oscillated by an oscillation circuit not illustrated is supplied to a primary winding of the output transformer Tr, and a drive signal for ultrasound output is generated in a secondary winding of the output transformer Tr.

The coil L2 is a coil for performing matching of impedance for the drive signal so as to be able to supply the drive signal efficiently to the ultrasound transducer 104. A current I₁ which is generated in the output transformer Tr, and a current I₁+I' which is a total of the current I₁ which is generated in the output transformer Tr and a reactive current I' which is generated in the coil L2 is supplied to the hand piece 102 via the aforementioned cable 2a.

Of the current I₁+I' which is supplied to the hand piece 102, the reactive current I' is diverted to the capacitor C2, and the current I₁ is supplied to the series resonance circuit configured by the resistor R1, the capacitor C1 and the coil L1. In this manner, the current I₁ which is generated in the output transformer Tr is supplied to the series resonance circuit. The series resonance circuit resonates in accordance with the value of the current I₁, and the ultrasound transducer 104 ultrasound-vibrates.

The conventional ultrasound operation system 101 cannot regulate the current I₁ which is generated in the output transformer Tr. Therefore, an amplitude value of a treatment portion varies depending on a component variation or an assembly variation of the ultrasound transducer 104, or a component variation or an assembly variation of the ultrasound probe. Further, in order to regulate the current I₁ which is generated in the output transformer Tr, the impedance at a drive time of the ultrasound transducer 104 needs to be sensed and fed back to the main apparatus, and a control program and a control section for performing feedback control need to be provided in the main apparatus.

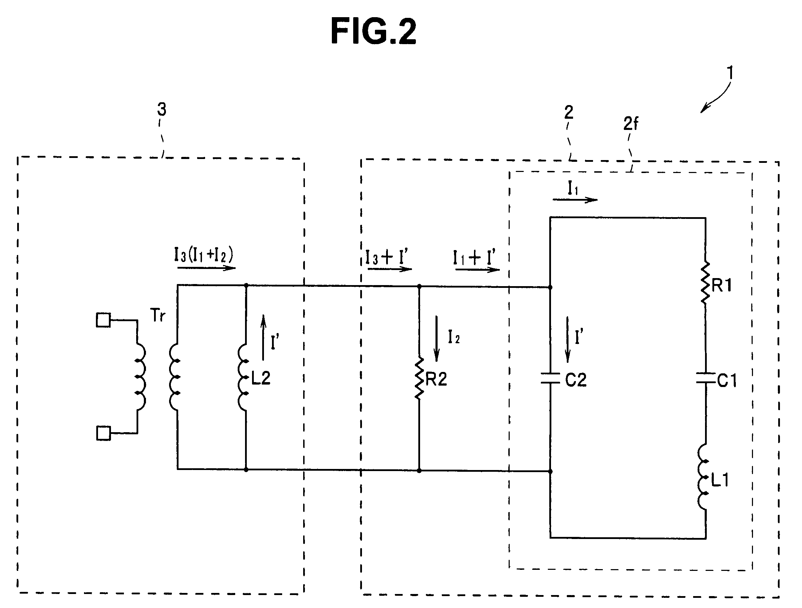

Fig. 2 is a diagram showing an equalizing circuit of the ultrasound operation system before ultrasound output of the present embodiment. In Fig. 2, the same components as in Fig. 10 are assigned with the same reference numerals and characters, and the description thereof will be omitted.

As shown in Fig. 2, the ultrasound operation system 1 is provided with a non-inductive resistor R2 in parallel between the output transformer Tr and the ultrasound transducer 2f. The non-inductive resistor R2 as an amplitude regulating portion diverts a current which is supplied to the ultrasound transducer 2f from the output transformer Tr as a drive current generating portion. Further, the non-inductive resistor R2 is provided at the hand piece 2 side, and the value of the non-inductive resistor R2 is the value substantially the same as a value of the resistor R1. The non-inductive resistor R2 as the amplitude regulating portion is provided at the hand piece 2 side, but may be provided at the main apparatus 3 side. Further, the amplitude regulating portion may be a resistor instead of the non-inductive resistor R2.

A value of a current I₃ of the output transformer Tr is determined so that when a current I₂ is diverted to the non-inductive resistor R2, the current I₁ flows into the ultrasound transducer 2f. More specifically, the value of the current I₃ is a value which is obtained by addition of a value of the current I₁ and a value of the current I₂. Whereby, the series resonance circuit resonates in accordance with the value of the current I₁, and the ultrasound transducer 2f ultrasound-vibrates.

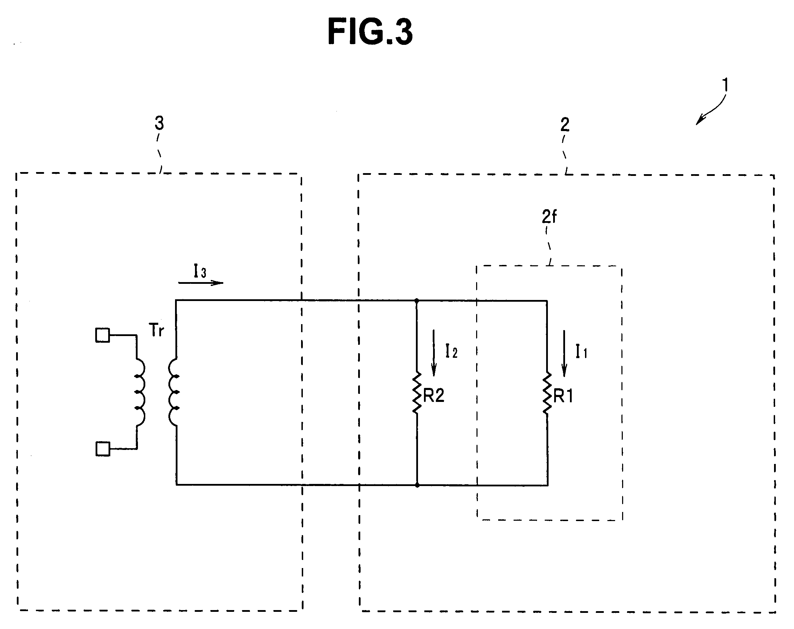

Fig. 3 is a diagram showing an equalizing circuit of the ultrasound operation system during ultrasound output of the present embodiment.

In the ultrasound operation system 1, the capacitor C1 and the coil L2, and the capacitor C2 and the coil L2 cancel out each other in the resonating state during ultrasound output. Accordingly, the equalizing circuit of the ultrasound operation system 1 has only the non-inductive resistor R2 and the resistor R1 of the ultrasound transducer 2f. The current I₁ which flows into the resistor R1 is converted into ultrasound vibration, and a distal end portion of the ultrasound probe 2d vibrates with a predetermined amplitude value.

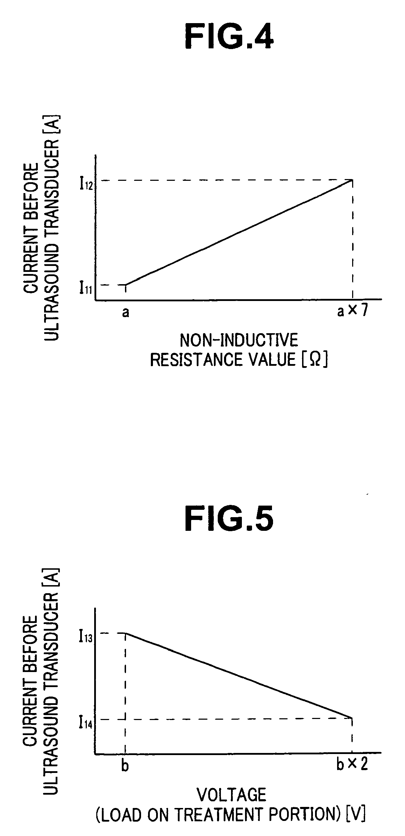

Here, the current I₁ which is supplied to the ultrasound transducer 2f will be described with use of Figs. 4 and 5.

Fig. 4 is an explanatory diagram for explaining a relationship of the non-inductive resistance value and the current which is supplied to the ultrasound transducer.

As shown in Fig. 4, when the value of the non-inductive resistor R2 increases to a×7[Ω] from a[Ω], the value of the current I₁ which is supplied to the resistor R1 of the ultrasound transducer 2f increases to I₁₂[A] from I₁₁[A]. This is because the current I₂ which is diverted to the non-inductive resistor R2 decreases as the value of the non-inductive resistor R2 becomes larger. More specifically, when the value of the non-inductive resistor R2 is large, the current I₂ which is diverted to the non-inductive resistor R2 decreases, and therefore, the current I₁ which flows into the resistor R1 of the ultrasound transducer 2f increases. Meanwhile, when the value of the non-inductive resistor R2 is small, the current I₂ which is diverted to the non-inductive resistor R2 increases, and therefore, the current I₁ which flows into the resistor R1 of the ultrasound transducer 2f decreases.

Fig. 5 is an explanatory diagram for explaining a relationship of a load on the treatment portion and a current which is supplied to the ultrasound transducer. In an example of Fig. 5, the non-inductive resistor R2 is fixed to a predetermined value, and the load (voltage) on the treatment portion 2e is changed. The load on the treatment portion 2e is equivalent to the load on the ultrasound transducer 2f. More specifically, increase in the load on the treatment portion 2e is equivalent to increase in the value of the resistor R1 of the ultrasound transducer 2f, and decrease in a load on the treatment portion 2e is equivalent to decrease in the value of the resistor R1 of the ultrasound transducer 2f.

As shown in Fig. 5, when the load on the treatment portion 2e increases to bx2[V] from b[V], the value of the current I₁ which is supplied to the resistor R1 of the ultrasound transducer 2f decreases to I₁₄[A] from I₁₃[A]. This is because the value of the resistor R1 becomes larger as the load on the treatment portion 2e becomes larger, and the current I₁ which is diverted to the resistor R1 decreases.

As above, when the non-inductive resistor R2 is fixed to the predetermined value, the current which flows into the ultrasound transducer 2f is regulated in accordance with the load on the treatment portion 2e, and the amplitude value of the ultrasound transducer 2f can be regulated.

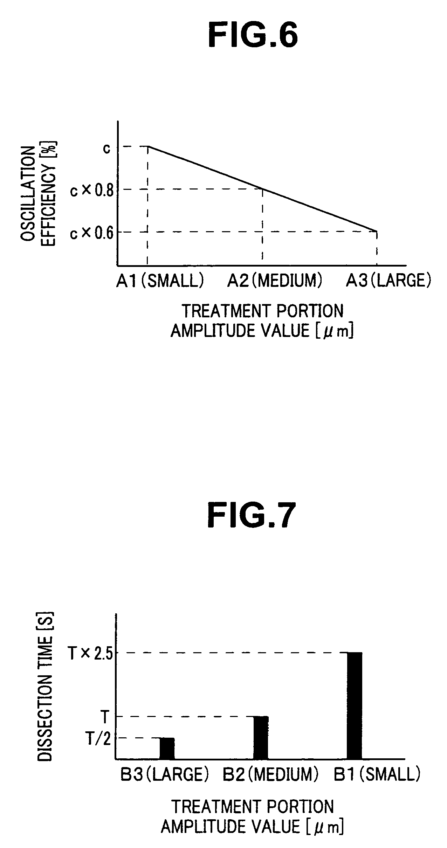

Here, the technical problem during treatment will be described with use of Figs. 6 to 9.

Fig. 6 is an explanatory diagram for explaining a relationship of the treatment portion amplitude value and the oscillation efficiency of the main apparatus.

When the amplitude value of the treatment portion 2e is A₂ which is a target (hereinafter, also called a central value), the oscillation efficiency of the main apparatus 3 is c×0.8[%]. When the amplitude value of the treatment portion 2e is A₁ which is smaller than the central value, the oscillation efficiency of the main apparatus 3 is c[%]. When the amplitude value of the treatment portion 2e is A₃ which is larger than the central value, the oscillation efficiency of the main apparatus 3 is c×0.6[%].

As above, when the amplitude value of the treatment portion 2e changes by about several tens µm from A₁ to A₃, the oscillation efficiency of the main apparatus 3 is reduced by about 40%. When the amplitude value of the treatment portion 2e becomes large, the oscillation efficiency of the main apparatus 3 is reduced. When the amplitude value of the treatment portion 2e further becomes larger to be an amplitude value outside a certain specified range, the oscillation efficiency of the main apparatus 3 is further reduced and ultrasound output cannot be performed.

Fig. 7 is an explanatory diagram for explaining a relationship of the amplitude value of the treatment portion and a dissection time. The dissection time of Fig. 7 is a time which is taken when a certain tissue is dissected by 20 cm.

As shown in Fig. 7, when the amplitude value of the treatment portion 2e is B₂ which is a central value, the dissection time is T[S]. When the amplitude value of the treatment portion 2e is B₁ which is smaller than the central value, the dissection time is Tx2.5[S] which is about 2.5 times as large as T[S]. When the amplitude value of the treatment portion 2e is B₃ which is larger than the central value, the dissection time is T/2[S] which is about 1/2 of T[S].

As above, when the amplitude value of the treatment portion 2e decreases, the dissection speed decreases, and the treatment time becomes long. Meanwhile, when the amplitude value of the treatment portion 2e is large, the dissection speed increases, but the stress of the ultrasound probe increases.

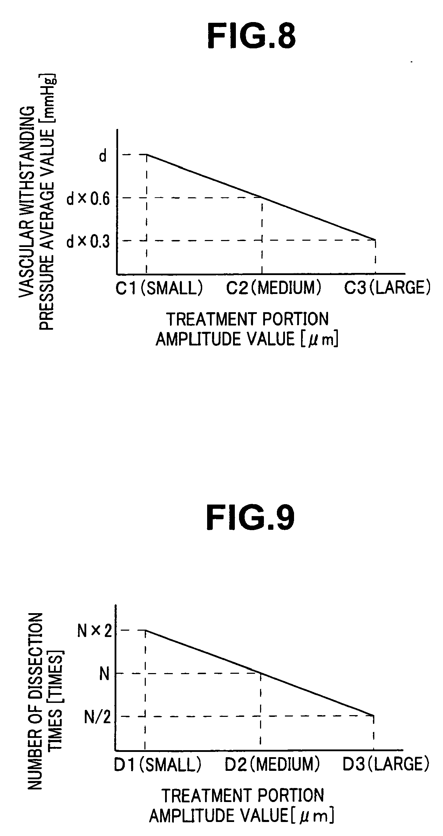

Fig. 8 is an explanatory diagram for explaining a relationship of the treatment portion amplitude value and a vascular withstanding pressure average value.

When the amplitude value of the treatment portion 2e is C₂ which is a central value, the vascular withstanding pressure average value is about d×0.6 [mmHg]. When the amplitude value of the treatment portion 2e is C₁ which is smaller than the central value, the vascular withstanding pressure average value is about d[mmHg]. When the amplitude value of the treatment portion 2e is C₃ which is larger than the central value, the vascular withstanding pressure average value is about d×0.3 [mmHg].

As above, when the amplitude value of the treatment portion 2e increases, the vascular withstanding pressure ability is reduced by about 70%.

Fig. 9 is an explanatory view for explaining a relationship of the treatment portion amplitude value and the number of dissection times.

As shown in Fig. 9, when the amplitude value of the treatment portion 2e is D₂ which is a central value, the number of dissection times is N. When the amplitude value of the treatment portion 2e is D₁ which is smaller than the central value, the number of dissection times is 2N which is twice as compared with the dissection times when the amplitude value is the central value. When the amplitude value of the treatment portion 2e is D₃ which is larger than the central value, the number of dissection times becomes N/2 which is 1/2 times as compared with the dissection times when the amplitude value is the central value.

As above, when the amplitude value of the treatment portion 2e increases, the number of dissection times decreases. This is because when the amplitude value of the treatment portion 2e increases, the Teflon pad of the treatment portion 2e easily wears. When the Teflon pad of the treatment portion 2e reaches a failure mode in which the Teflon pad is worn, the ultrasound probe 2d and the movable piece are in contact with each other, and a crack occurs to the ultrasound probe 2d.

Here, control of the amplitude value of the treatment portion 2e in the case of the amplitude value of the treatment portion 2e varying due to a component variation or the like of the ultrasound transducer 2f or the like will be described.

When the amplitude value of the treatment portion 2e is around the central value, the load on the treatment portion 2e is around the central value. In this case, the value of the resistor R1 of the ultrasound transducer 2f does not vary, and the current I₂ which is diverted to the non-inductive resistor R2 and the current I₁ which flows into the resistor R1 do not vary. Since the current I₁ which flows into the resistor R1 does not vary like this, the amplitude value of the treatment portion 2e does not vary.

When the amplitude value of the treatment portion 2e is lower than a value around the central value, the load on the treatment portion 2e becomes small. This case is equivalent to the value of the resistor R1 of the ultrasound transducer 2f becoming small, and the current I₂ which is diverted to the non-inductive resistor R2 decreases, whereas the current I₁ which flows to the resistor R1 increases.

When the amplitude value of the treatment portion 2e is lower than a value around the central value, the problem of reducing the dissection speed arises. However, when the amplitude value of the treatment portion 2e is lower than the value around the central value like this, the current I₁ which flows into the resistor R1 increases, and therefore, the amplitude value of the treatment portion 2e increases, and reduction in the dissection speed can be suppressed.

When the amplitude value of the treatment portion 2e is higher than a value around the central value, the load on the treatment portion 2e becomes large. This case is equivalent to the value of the resistor R1 of the ultrasound transducer 2f becoming large, and the current I₂ which is diverted to the non-inductive resistor R2 increases, whereas the current I₁ which flows into the resistor R1 decreases.

When the amplitude value of the treatment portion 2e is higher than a value around the central value, there arise the problems of reduction in a vascular withstanding pressure ability, promotion of wear of the Teflon pad and increases in stress of the ultrasound probe. However, when the amplitude value of the treatment portion 2e is higher than a value around the central value like this, the current I₁ which flows into the resistor R1 decreases. Therefore, the amplitude value of the treatment portion 2e decreases, reduction in the vascular withstanding pressure ability, promotion of wear of the Teflon pad and increase in stress of the ultrasound probe can be suppressed.

Next, control of the amplitude value of the treatment portion 2e in the case in which the grasping force amount of the handle 2c varies due to a component variation or the like of the handle 2c will be described.

When the grasping force amount is around the central value, the load on the treatment portion 2e is around the central value. In this case, the value of the resistor R1 of the ultrasound transducer 2f does not vary, and the current I₂ which is diverted to the non-inductive resistor R2 and the current I₁ which flows into the resistor R1 do not vary. Accordingly, the current I₁ which flows into the resistor R1 does not vary, and therefore, the amplitude value of the treatment portion 2e does not vary.

When the grasping force amount is lower than a value around the central value, the load on the treatment portion 2e becomes small. This case is equivalent to the value of the resistor R1 of the ultrasound transducer 2f becoming small, and the current I₂ which is diverted to the non-inductive resistor R2 decreases, whereas the current I₁ which flows into the resistor R1 increases.

When the grasping force amount is lower than a value around the central value, there arises the problem of reducing the dissection speed. However, since when the grasping force amount is lower than a value around the central value, the current I₁ which flows into the resistor R1 increases, the amplitude value of the treatment portion 2e increases, and reduction in the dissection speed can be suppressed.

When the grasping force amount is higher than a value around the central value, the load on the treatment portion 2e becomes large. This case is equivalent to the value of the resistor R1 of the ultrasound transducer 2f becoming large, the current I₂ which is diverted into the non-inductive resistor R2 increases, whereas the current I₁ which flows into the resistor R1 decreases.

When the grasping force amount is higher than a value around the central value, there arise the problems of promotion of wear of the Teflon pad, and increase in stress of the ultrasound probe. However, since when the grasping force amount is higher than a value around the central value like this, the current I₁ which flows into the resistor R1 decreases, the amplitude value of the treatment portion 2e decreases, and promotion of the wear of the Teflon pad and increase in stress of the ultrasound probe can be suppressed.

Next, control of the amplitude value of the treatment portion 2e in the case of a hardness of a site to be treated varying will be described.

When the site to be treated is an ordinary tissue such as a vessel, the load on the treatment portion 2e becomes a value around the central value. In this case, the value of the resistor R1 of the ultrasound transducer 2f does not vary, and the current I₂ which is diverted to the non-inductive resistor R2 and the current I₁ which flows into the resistor R1 do not vary. Accordingly, the current I₁ which flows into the resistor R1 does not vary, and therefore, the amplitude value of the treatment portion 2e does not vary.

When the site to be treated is a thin tissue such as a mesenterium, the load on the treatment portion 2e becomes small. This case is equivalent to decrease in the value of the resistor R1 of the ultrasound transducer 2f, and the current I₂ which is diverted to the non-inductive resistor R2 decreases, whereas the current I₁ which flows into the resistor R1 increases.

When the site to be treated is a thin tissue such as a mesenterium, there arises the problem that the dissection speed is reduced. However, since when the site to be treated is a thin tissue such as a mesenterium like this, the current I₁ which flows into the resistor R1 increases, the amplitude value of the treatment portion 2e increases, and reduction in the dissection speed can be suppressed.

When the site to be treated is a hard tissue such as a uterine ligament, the load on the treatment portion 2e becomes large. This case is equivalent to increase in the value of the resistor R1 of the ultrasound transducer 2f, and the current I₂ which is diverted to the non-inductive resistor R2 increases, whereas the current I₁ which flows into the resistor R1 decreases.

When the site to be treated is a hard tissue such as a uterine ligament, there arises the problem that the main apparatus 3 cannot output ultrasound since the stress increase and the load of the ultrasound probe are large. However, since when the site to be treated is a hard tissue such as a uterine ligament like this, the current I₁ which flows into the resistor R1 decreases, the amplitude value of the treatment portion 2e decreases, and inability of the main apparatus 3 to output ultrasound due to large stress increase and load of the ultrasound probe can be suppressed.

As above, in the ultrasound operation system 1 of the present embodiment, the non-inductive resistor R2 is provided in parallel between the output transformer Tr of the main apparatus 3 and the ultrasound transducer 2f in the hand piece 2, whereby the current which is supplied to the ultrasound transducer 2f from the main apparatus 3 is diverted. Whereby, when the load on the treatment portion 2e becomes large, the current which flows into the non-inductive resistor R2 increases, and the current which flows into the ultrasound transducer 2f decreases. Further, when the load on the treatment portion 2e becomes small, the current which flows into the non-inductive resistor R2 decreases, and the current which flows into the ultrasound transducer 2f increases.

As a result, when the load on the treatment portion 2e becomes large, the ultrasound operation system 1 decreases the amplitude value of the treatment portion 2e, whereas when the load on the treatment portion 2e becomes small, the ultrasound operation system 1 can increase the amplitude value of the treatment portion 2e. More specifically, even when the load on the treatment portion 2e varies, the ultrasound operation system 1 can control the amplitude value of the treatment portion 2e so that the amplitude value is close to the central value.
As above, the ultrasound operation system 1 diverts the current which is outputted from the output transformer Tr, and can increase or decrease the current which flows into the ultrasound transducer 2f in accordance to the load on the treatment portion 2e, by the non-inductive resistor R2 provided in the hand piece 2. Whereby, the ultrasound operation system 1 can automatically regulate the amplitude value in accordance with the load on the treatment portion 2e.
As a result, the ultrasound operation system 1 of the present embodiment does not need to provide a control program and a control section for feedback in the main apparatus, does not need to feed back impedance from the ultrasound transducer, and perform feedback control of controlling the current which flows into the ultrasound transducer.
Consequently, according to the ultrasound operation system 1 of the present embodiment, the current which flows into the ultrasound transducer can be controlled without the need for the control program and the control section for performing feedback control.
The present invention is not limited to the aforementioned embodiment, and various changes, modifications and the like can be made within the range without departing from the gist of the present invention.

## Claims

1. An ultrasound operation system (1), comprising:
a drive current generating portion (Tr);
a surgical treatment instrument (2) comprising an object to be driven including an ultrasound transducer (2f) which is configured to be driven by the drive current generating portion (Tr), and a treatment portion (2e) which is connected to the ultrasound transducer (2f) to perform treatment of a living tissue by an ultrasound vibration generated in the ultrasound transducer (2f); and
**characterized in that** the ultrasound operation system (1) further comprises:
a non-inductive resistor (R2) which is provided in parallel between the drive current generating portion (Tr) and the object to be driven, and is configured to perform regulation of an amplitude of the ultrasound transducer by diverting a current which flows into the object to be driven from the drive current generating portion (Tr) in accordance with a state of a load exerted on the treatment portion (2e) and regulating an amount of a current which flows into the object to be driven.

2. The ultrasound operation system according to claim 1,
wherein the non-inductive resistor (R2) is provided in the surgical treatment instrument (2).

3. The ultrasound operation system according to claim 2,
wherein a value of the non-inductive resistor (R2) is substantially the same as a value of a resistor of the ultrasound transducer (2f) which the object to be driven has.

## Patentansprüche

1. Ultraschallbetätigungssystem (1), das umfasst:
ein Antriebsstromerzeugungsabschnitt (Tr);
ein chirurgisches Behandlungsinstrument (2), das ein anzutreibendes Objekt mit einem Ultraschallwandler (2f), der dazu eingerichtet ist, von dem Antriebsstromerzeugungsabschnitt (Tr) angetrieben zu werden, und einen Behandlungsabschnitt (2e) umfasst, der mit dem Ultraschallwandler (2f) verbunden ist, um eine Behandlung eines lebenden Gewebes durch eine in dem Ultraschallwandler (2f) erzeugte Ultraschallschwingung durchzuführen; und
**dadurch gekennzeichnet, dass** das Ultraschallbetätigungssystem (1) ferner umfasst:
einen nicht-induktiven Widerstand (R2), der parallel zwischen dem Antriebsstromerzeugungsabschnitt (Tr) und dem anzutreibenden Objekt angeordnet ist und der dazu eingerichtet ist, eine Regulierung einer Amplitude des Ultraschallwandlers durchzuführen, indem er einen Strom, der in das von dem Antriebsstromerzeugungsabschnitt (Tr) anzutreibende Objekt fließt, in Übereinstimmung mit einem auf den Behandlungsabschnitt (2e) wirkenden Lastzustand ablenkt und einen Strombetrag reguliert, der in das anzutreibende Objekt fließt.

2. Ultraschallbetätigungssystem gemäß Anspruch 1,
bei dem der nicht-induktive Widerstand (R2) in dem chirurgischen Behandlungsinstrument (2) vorgesehen ist.

3. Ultraschallbetätigungssystem gemäß Anspruch 2,
bei dem ein Wert des nicht-induktiven Widerstands (R2) im Wesentlichen gleich einem Wert eines Widerstands des Ultraschallwandlers (2f) ist, den das anzutreibende Objekt hat.

## Revendications

1. Système (1) d'opération par ultrasons, comprenant :
une partie (Tr) de génération de courant d'entraînement ;
un instrument (2) de traitement chirurgical comprenant un objet à entraîner incluant un transducteur à ultrasons (2f) qui est configuré pour être entraîner par la partie (Tr) de génération de courant d'entraînement, et une partie de traitement (2e) qui est connectée au transducteur à ultrasons (2f) pour exécuter un traitement d'un tissu vivant par une vibration ultrasonore générée dans le transducteur à ultrasons (2f) ; et
**caractérisé en ce que** le système (1) d'opération par ultrasons comprend en outre :
une résistance non inductive (R2) qui est prévue en parallèle entre la partie (Tr) de génération de courant d'entraînement et l'objet à entraîner, et est configurée pour exécuter une régulation d'une amplitude du transducteur à ultrasons en détournant un courant qui pénètre dans l'objet à entraîner depuis la partie (Tr) de génération de courant d'entraînement en fonction d'un état d'une charge exercée sur la partie de traitement (2e) et en régulant une quantité d'un courant qui pénètre dans l'objet à entraîner.

2. Système d'opération par ultrasons selon la revendication 1,
dans lequel la résistance non inductive (R2) est prévue dans l'instrument (2) de traitement chirurgical.

3. Système d'opération par ultrasons selon la revendication 2,
dans lequel une valeur de la résistance non inductive (R2) est sensiblement la même qu'une valeur d'une résistance du transducteur à ultrasons (2f) qu'a l'objet à entraîner.
